# EUROPEAN PATENT SPECIFICATION

(11) **EP 2 501 321 B1**
(45) Date of publication and mention of the grant of the patent: **12.10.2016**
(21) Application number: 10803136.0
(22) Date of filing: 18.11.2010
(51) Int. Cl.: A61B 50/00, A61B 50/30

(54) **DEVICE TO TRANSPORT MEDICAL INSTRUMENTS**
VORRICHTUNG FÜR DEN TRANSPORT VON MEDIZINISCHEN INSTRUMENTEN
DISPOSITIF POUR TRANSPORTER DES INSTRUMENTS MÉDICAUX

(30) Priority: 18.11.2009 IT UD20090207
(43) Date of publication of application: 26.09.2012
(73) Proprietor: Steelco Spa, 31039 Riese Pio X (IT)
(72) Inventor: ZARDINI, Fabio, I-31033 Castelfranco Veneto (IT)
(74) Representative: Petraz, Davide Luigi
(86) International application number: PCT/IB2010/002935
(87) International publication number: WO 2011/061600

(56) References cited:
- EP-A1- 0 580 971
- EP-A1- 1 477 106
- EP-A2- 0 103 753
- US-A- 4 753 367
- US-A- 5 938 646
- US-B1- 6 354 312

## Description

### FIELD OF THE INVENTION

The present invention concerns a device to transport medical instruments in a hospital or outpatient environment, in particular flexible endoscopes, both before and after use. In particular the device according to the present invention can be used to transport dirty medical instruments to a washing station where they are washed, and if necessary heat disinfected, and subsequently, to a drying station where they are dried ready to use again.

### BACKGROUND OF THE INVENTION

Flexible endoscopes are known, used to examine the internal parts of the human body or animals. Such endoscopes are normally of a diameter from 0.5 to 20 mm and from 300 to 4,000 mm long and are provided with internal channels, into which a surgical video acquisition device or a flow of fluid such as air or water is introduced. Because of their use, these endoscopes have to be washed and disinfected carefully after use.

In some cases, the washing station may be at a certain distance from the operating theater and this is why it is necessary to provide a container to transport instruments from one place to another, which minimizes and ideally completely prevents any risk both of contamination by the operators responsible and any accidental damage to the endoscope.

Documents EP-A-1.477.106, EP-A-0.580.971and US-B-6,354,312 disclose various known container for cleaning and sterilizing medical instruments.

Document US-A-5,938,646 discloses a known medical utility storage assembly for reusable medical equipment, including means for drying the medical equipment subjected to a cleaning cycle after use.

One purpose of the present invention is therefore to produce a container to transport medical instruments, particularly flexible endoscopes, before and after use, which allows a protection of the endoscope from contamination and damage before and after its use.

Another purpose is to produce a container which gives protection to the health workers, to the operators and to the patients from contamination and possible infection deriving from dirty medical instruments before and after use.

Another purpose is to produce a container which protects the unused medical instrument from so-called transverse contamination with other medical instruments or surfaces which are dirty or infected.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a device according to the present invention can be used to transport one or more medical instruments both before and after use, whether they are clean or dirty. The device comprises a container, a bag able to contain the medical instrument and a lid.

The container comprises lateral walls to define an internal containing seating and which have upper ends that delimit a first aperture through which the bag is inserted into the seating of the container.

According to one feature of the present invention, the upper ends of the lateral walls of the container comprise a first edge, inside or outside the seating and shaped to cooperate with a mating upper margin of the bag so as to define a selectively releasable attachment of the bag and the container.

In some forms of embodiment, the positioning of the first edge inside the containing seating increases the safety of the invention and reduces contamination, particularly when the medical instrument is deposited in the bag which, being well constrained and open thanks to the coupling with the first edge, does not constitute any obstruction or interference and eliminates potentially dangerous situations. In any case, if there are problems, such as the spilling of biological material during the course of moving the dirty medical instrument or in the attachment of the bag by the operator, it is guaranteed that possible contaminants always remain in the internal seating of the container.

In some forms of embodiment, the upper ends also comprise a second edge, in an external or respectively internal position to the first edge with respect to the internal seating, which second edge is able to cooperate with the lid to close the container.

In some forms of embodiment of the present invention, at least one of the lateral walls of the container have a second aperture which puts the outside in communication with the internal seating; the second aperture is provided with a rapid connector for the fluidic connection, on one side to blower means to blow in a drying fluid and on the other side, inside the container, by means of connectors, to the medical instrument. This form of embodiment is useful, for example, to maneuver, manipulate or access the medical instrument in the container, in order to connect an external drying device after washing, while keeping the instrument in any case inside the container.

Advantageously, the first edge is made in substantial correspondence to the summit of the lateral walls, inside the seating.

In some forms of embodiment, the second edge is also made in substantial correspondence to the summit of the lateral walls, in a position at a greater height than the first edge with respect to the bottom of the container.

In variant embodiments, at least the first edge develops completely along the perimeter of the first aperture.

In other variant embodiments, at least the first edge develops partially along the perimeter of the first aperture.

In some forms of embodiment, the first edge is localized in desired different positions of the upper ends along the perimeter of the first aperture, for example made as upward-protruding portions, protuberances, points or other in desired angular positions along the perimeter of the container.

In variant embodiments, at least the first edge has a beveled conformation in a curl, tongue or lip, advantageous for an easy coupling of the bag, in particular in forms of embodiment in which the upper margin of the bag has an enlarged edge, or tongues or other protuberances, either rigid or flexible.

In some forms of embodiment of the invention, the container also comprises a bottom wall from which the lateral walls extend and which includes a surface inside the seating from which protruding elements arise, able to function as spacers to keep the bag distanced from the bottom wall by a desired amount.

In advantageous forms of embodiment, the bag can be selectively closed reversibly by means of closing means, such as a through lace which acts as a drawstring, or other closing means such as Velcro, a zip or other, anyway associated with the upper margin of the bag. This increases the safety of the invention and prevention from contamination.

Advantageous forms of embodiment provide that the lid is provided with display means, for example of the disc selector type with chromatic signaling, which can be set on at least two different significant colors, able to selectively display at least the state of cleanliness or dirtiness of the medical instrument contained therein.

Advantageous forms of embodiment provide that the lid comprises an inclined peripheral portion, having an extension such as to completely cover the second edge of the upper ends of the lateral walls of the container when the lid is in the closed position, so as to prevent contacts between potentially contaminated zones of the container and the outside.

Another feature of the present invention concerns a container for one or more medical instruments to be transported both before and after they have been used, comprising lateral walls to define an internal containing seating and which have upper ends that delimit a first aperture through which a bag containing the medical instruments is inserted into the seating. The bag is conformed to be associated with the upper ends of the lateral walls. The upper ends comprise a first edge inside or outside the seating and shaped to cooperate with a mating upper margin of the bag so as to define a selectively releasable attachment of the bag and the container, and a second edge, in a position outside or respectively inside the first edge with respect to the internal seating, the second edge being able to cooperate with a mating lid in order to close the container.

In some forms of embodiment at least one of the lateral walls of the container has a second aperture which puts the outside in communication with the internal seating, the second aperture being provided with a rapid connector for the fluidic connection on one side to blower means to blow in a drying fluid, and on the other side, by means of connectors, to the medical instrument.

Another feature of the present invention concerns a bag to contain one or more medical instruments to be transported by means of a container both before and after use, and which can be inserted in the container. The bag according to the present invention comprises an upper margin which delimits a mouth through which the medical instrument is inserted. The upper margin is conformed to cooperate with a first internal edge of the container. The bag also contains closing means able to selectively close the mouth in a reversible manner.

A further feature of the present invention concerns a lid to close the container for one or more medical instruments to be transported both before and after use. The container comprises a peripheral portion able to cooperate with the upper ends of the container so as to close the container and prevent the contact of internal zones of the container that are potentially contaminated with the outside. The lid according to the present invention comprises, on its external surface, normally the visible surface when the lid closes the container, indicator or display means able to selectively indicate or display, either directly or indirectly, the state at least of dirtiness or cleanliness of the medical instrument contained in the container.

Furthermore, another feature of the present invention concerns a method for drying one or more medical instruments subjected to a cleaning cycle after use and inserted in a container used to transport the medical instruments both before and after use.

The drying method comprises a step of positioning the medical instrument inside a drying machine of a drying station where blower means are able to blow a drying fluid, typically sterile air, toward the medical instrument contained in the container in order to dry at least the external surface thereof, the blower means also being connected by means of at least a first connector element to a mating second connector element of the medical instrument so as to blow the drying fluid also inside the medical instrument.

The drying method according to the present invention provides to subject to drying, in the drying machine, the medical instrument disposed inside the same container which is used for transport. According to the invention moreover, the connection between the at least one first connector element and the mating second connector element of the medical instrument is carried out keeping the medical instrument inside the container, connecting the first connector element to a rapid connector associated with a corresponding lateral aperture made in the container, the rapid connector providing connections inside the container for connection to the second connector element of the medial instrument.

A further feature of the present invention concerns a method to transport one or more medical instruments both before and after use, by means of a transport device as described above, comprising:
- a first step in which the bag is positioned in the seating of the container and the upper margin of the bag is attached to the first internal or external edge, also determining the opening thereof;
- a second step in which the dirty medical instrument is disposed in the bag, the bag is closed in a reversible manner and the lid is positioned so as to close the container in cooperation with the second external or internal edge respectively;
- a third step in which the container is transported to a washing station where the lid is opened, the dirty medical instrument is removed so as to be subjected to a cleaning cycle, the used bag is eliminated and at the same time a new, clean bag is positioned in the seating of the container, as in the first step;
- a fourth step in which the medical instrument is positioned in the new, clean bag and is transported to a drying station where it is subjected to drying in accordance with the drying method described above, temporarily removing the new bag, or leaving it inside the container;
- a fifth step in which the new bag containing the dried medical instrument is closed in a reversible manner and the lid is positioned so as to close the container as in the second step, making the medical instrument available for another use.

In some forms of embodiment of the invention, the cleaning cycle can provide a pre-wash, for example with cold water and/or ultrasound, and a subsequent wash.

In other forms of embodiment of the invention, the wash can include, or be combined with, a chemical disinfection.

In other forms of embodiment, the wash can include, or be combined with, a heat disinfection.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a perspective representation of a device according to the present invention;
- fig. 2 is a section of part of the device in fig.1;
- fig. 3 is a section of a variant of the device in fig.1;
- fig. 4 is a section of another variant of the device in fig.1;
- fig. 5 is a section of a further variant of the device in fig.1;
- fig. 6 is a schematic representation of a cleaning cycle according to the invention;
- fig. 7 is a schematic representation of a method to transport a medical instrument;
- fig. 8 is a plane view from above of a variant of the device according to the present invention;
- fig. 9 is a lateral view from the direction of the arrow A in fig. 8;
- fig. 10 is a lateral view from the direction of the arrow B in fig. 8;
- fig. 11 is a section along the line XI - XI in fig. 8;
- fig. 12 shows a part of fig. 11 and a relative enlarged detail;
- fig. 13 is a part of a section along the line XIII - XIII in fig. 8 and of two relative enlarged details;
- fig. 14 is a part of a section along the line XIV - XIV in fig. 8;
- fig. 15 is a plane view from above of a lid of the device in fig. 8;
- fig. 16 is a section along the line XVI - XVI in fig. 15.

To facilitate comprehension, the same reference numbers have been used, where possible, to identify common elements in the drawings that are identical. It is understood that elements and characteristics of one form of embodiment can conveniently be incorporated into other forms of embodiment without further clarifications.

### DETAILED DESCRIPTION OF A PREFERENTIAL FORM OF

### EMBODIMENT

With reference to the attached drawings, a device 10 according to the present invention can be used to transport a medical instrument, such as a wrapped flexible endoscope, before and after its use.

The device 10 comprises a box-like container 12, advantageously made of a rigid or semi-rigid material, for example plastic, able to be re-used or made of a material such as to support a repeated number of heat disinfection cycles.

According to one form of embodiment, the container 12 is advantageously made by means of molding a plastic material, such as for example ABS.

The container 12 is formed by a bottom wall 20, generally flat, from which four lateral walls 14 extend one after the other, substantially at a right angle so as to define, in this case, a plane rectangular shape, for example slightly tapered toward the bottom. It is clear that the rectangular shape is not to be understood as restrictive of the present invention.

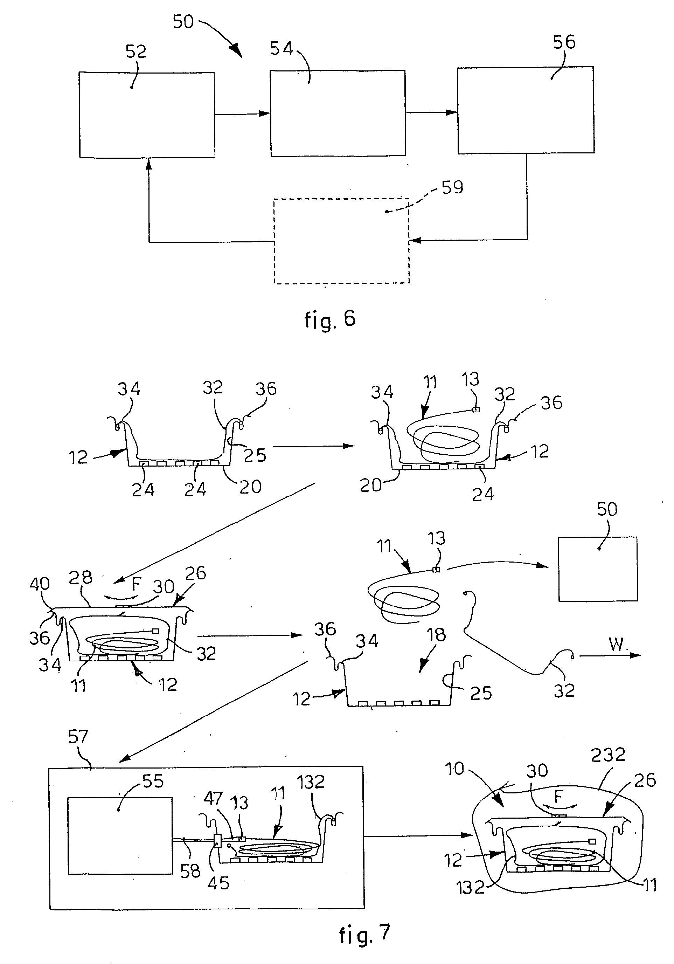

The lateral walls 14 and the bottom wall 20 delimit a seating 25 in which the medical instrument 11 to be transported is positioned (fig. 7) and which has previously been inserted into a bag 32 which will be described in detail hereafter in the description.

The dimensions of the bottom wall 20 are such as to allow to deposit a wrapped endoscope in the seating 25 without stress or unwanted excessive folds or deformations.

Dimensions of the container 12 are for example about 400 - 600 mm in length, about 300 - 500 mm in width and about 50 - 200 mm in height.

The lateral walls 14 and the bottom wall 20 have a preferential thickness comprised between 3 and 6 mm.

In order not to damage the medical instrument 11, it is preferable that all the edges, the intersections between the walls and the corners of the container 12 are curved and beveled. This is further advantageous because it eliminates zones or niches where bacteria or pathogens in general can grow and proliferate, or dirt and contaminants can accumulate.

The container 12 has upper ends 16 made at least partly around the lateral walls 14, in this case along all the upper perimeter of the four lateral walls 14.

The four upper ends 16 delimit a first upper aperture 18, through which the medical instrument 11 in the bag 32 can be inserted and positioned in the seating 25.

The bottom wall 20 has a surface 22 inside the container 12, on which protruding elements or studs 24 are made, protruding upward by a determined amount, which function as spacers to keep the medical instrument 11 separate from the bottom wall 20.

The device 10 also comprises a lid 26 able to cooperate with the upper ends 16 of the walls 14 in order to close the first aperture 18 of the container 12 and protect the operator from contamination.

In some forms of embodiment, on its outside or upper surface 28, the lid 26 has an indicator element conformed for example, as a selector disc 30 able to be driven in rotation (as shown by the arrows F in fig. 7) so as to present to view a first region colored red, if there is a dirty instrument in the container 12, or a second region colored green if there is a clean instrument in the container 12.

In further forms of embodiment, the indicator element can be formed by or associated with a bar code or a RFID tag which memorizes similar or further information.

The device 10 also comprises a bag 32 made of flexible material, advantageously plastic, for example polyethylene, preferably high density polyethylene (HDPE), able to be easily inserted in the seating 25, adapting itself to the required forms and dimensions thanks to its flexibility.

The bag 32 comprises a containing body 31, formed by lateral walls 31a and a bottom wall 31b. The lateral walls 31a have an upper margin 35 which delimits an aperture, or mouth, 35a of a shape substantially mating with that of the first aperture 18, in this case, therefore, of a rectangular shape, and through which the medical instrument 11, for example a wrapped endoscope, can be inserted into the bag 32. The bag 32 thus acts advantageously as a covering or cover in order to protect the container 12 and the operator from contact with the dirty medical instrument.

In some forms of embodiment of the invention, the bag 32 comprises a selectively reversible closing mean, advantageously associated with the upper margin 35, by means of which the bag 32 can be closed.

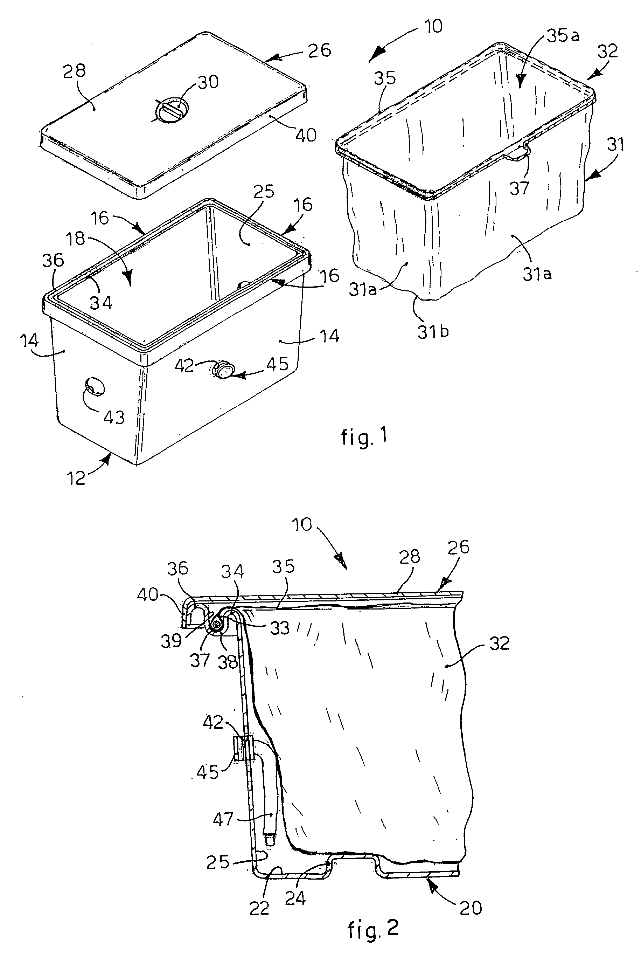

In some forms of embodiment of the invention, the closing mean comprises an eyelet 33 made along the perimeter of the upper margin 35 of the mouth 35a of the bag 32, inside which there is a through and sliding lace 37, with two free ends, or one free end and the other constrained, which acts as a drawstring and can be used by the user to pull the mouth 35a together and thus close the bag 32 (figs. 1 and 2).

Each of the upper ends 16 of the walls 14 of the container 12 comprises a first edge 34, conformed as a curl, lip or tongue or in any case an upside down "U", and made inside the seating 25, and a second edge 36, this also conformed as a curl, lip or tongue or in any case an upside down "U", facing toward the outside with respect to the seating 25.

According to the construction of the container 12, the second edge 36 is disposed outside the first edge 34 with respect to the seating 25 (fig. 2).

The first edge 34 is connected to the second edge 36 by a curved portion 38, of an opposite shape to the first edge 34 and second edge 36. The curved connection portion 38 defines a peripheral groove 39 which acts as a positioning seating for the upper margin 35 of the bag 32 (fig. 2) or for one of its enlarged or rigid connection portions.

The extension toward the outside of the curved portion 38, or its radius of curvature, is such as to distance the first internal edge 34 from the second external edge 36 by a desired amount.

The dimensions of the lid 26 are mating with those of the second edge 36, so that, when it is coupled to the container 12, the lid 26 completely covers the second external edge 36 and, even more so, the first internal edge 34, thus constituting a protection from contamination. In the form of embodiment shown, the lid 26 has a peripheral portion or edge 40 inclined downward, to give a greater covering of the first edge 36 for the purposes of cleaning, to prevent contamination and to facilitate the coupling to the container 12 for the purposes of closing it (fig. 2).

The upper margin 35 of the bag 32 is enlarged by the presence of the lace 37 inside it and this enlargement is advantageously used, in some forms of embodiment of the invention, to achieve a stable attachment of the bag 32 in the groove 39 between the first edge 34 and the second edge 36. In particular the upper margin 35 is positioned astride the second edge 34, so that the eyelet 33 and the lace 37 are, at least partly, positioned in the groove 39, advantageously with a determinate coupling by interference between the walls of the groove 39 and the upper margin 35 of the bag 32.

In advantageous forms of embodiment, at least one of the lateral walls 14 of the container 12 has a second through aperture 42, to which a rapid connector 45 is associated which allows the communication between the environment outside the container 12 and the seating 25 inside the container 12 (figs. 1 and 2).

The rapid connector 45 has connections 47 on the inner side of the container 12, for example disposed in a suitable compartment, by means of which it can be advantageously used to connect the medical instrument 11 to the inside of the container 12 with possible external devices, such as tubes or connectors which blow in a fluid, for example for the purpose of drying or other.

Moreover, one or more of the lateral walls 14 can have third apertures 43 through which, as will be explained hereafter, drying air is made to pass, which has been made sterile by a suitable filter, toward the inside of the container 12, which is especially advantageous in the case where there is the lid 26 closing the container 12; the air is then discharged outside.

In some forms of embodiment of the invention, the first internal edge 34 could also have a eyelet, made partly or completely on the whole perimeter of the upper ends 16 of the container 12, into which the upper margin 35 of the bag 32 is inserted and constrained, possibly by means of rapid closing means such as Velcro or a zip.

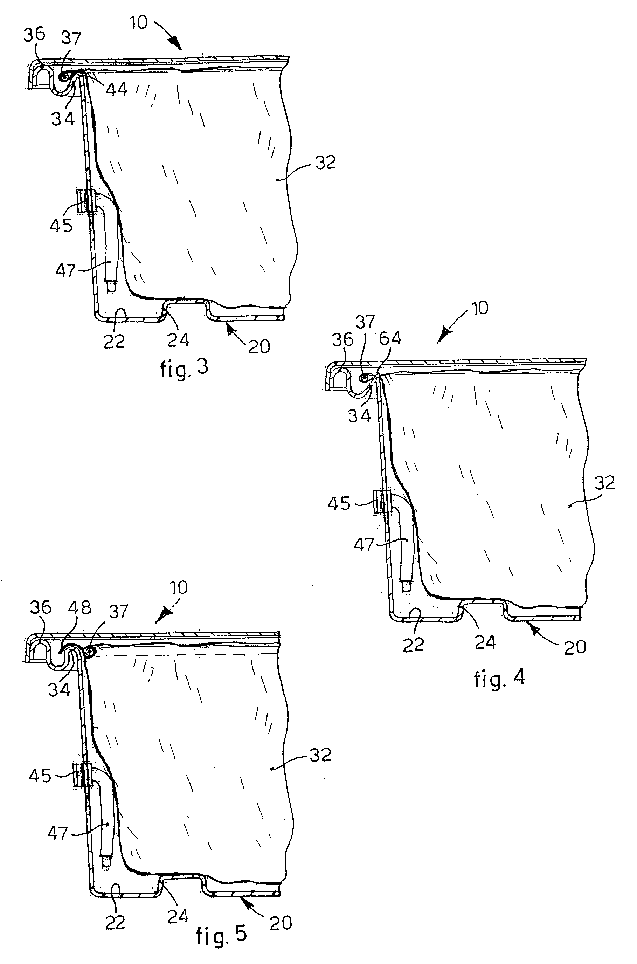

A variant of the invention (fig. 3) can provide that the first edge 34 has upward facing protuberances 44 disposed localized in desired angular positions, for example at the four peripheral corners of the first edge 34 in the case of a rectangular shape.

Another variant (fig. 4) provides that the first edge 34 has upward facing points 64, again disposed localized in desired angular positions, for example at the four outside corners of the first edge 34 in the case of a rectangular shape.

The bag 32 can therefore be constrained to the container by anchoring to the protuberances 44 or to the points 64, advantageously perforating at desired points the flexible material of which the upper margin 35 of the bag 32 is made, in particular in correspondence to the four corners.

Advantageously, in order to stop the bag 32 from ripping with the weight of the medical instrument 11 contained therein, the upper margin 35 of the bag 32 can have reinforcements, for example in the form of a thickening or fold of the material, at least in a position mating to that of the protuberances 44.

Another variant of the invention (fig. 5) provides that the bag 32 comprises curved portions or tongues 48 made of a rigid material, such as rigid plastic, protruding toward the outside with respect to the mouth or aperture 35a of the bag, for example disposed at the four corners, having a shape mating with that of the first edge 34 of the container 12 and by means of which it is possible to attach the bag 32 in the groove 39 between the first edge 34 and the second edge 36. In this case, the lace 37 will be in an inside position with respect to the curved tongues 38 in order to allow the bag 32 to be closed.

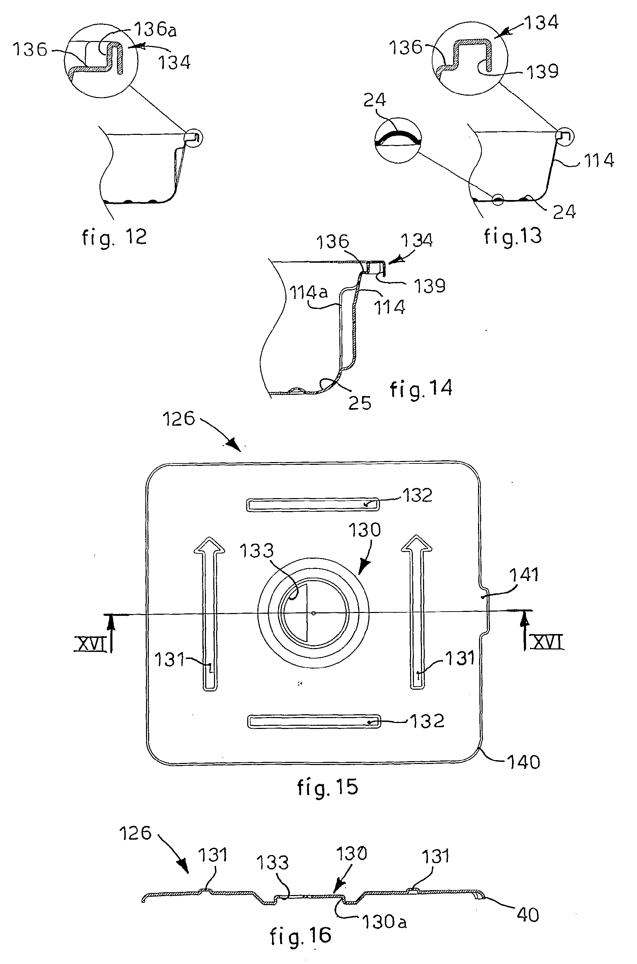

The drawings 8 to 16 show a variant of a form of embodiment of the device according to the present invention, shown by the reference number 110, comprising a container 112 and a lid 126, shown for convenience only in figs. 15 and 16, even if the lid 26 as in figs. 1-5 could be used.

The container 112 has lateral walls 114 which extend from the bottom 20, defining the seating 25. In this solution too, the protruding elements 24 can be provided on the bottom 20, with the same function as above.

The lateral walls 114 have upper ends 116, in this case along all of the upper perimeter of the four lateral walls 14, which delimit the first aperture 18. In this case, the lateral walls 114 also have third apertures or slits 143 for the passage of the drying air.

Each of the upper ends 116 of the walls 114 of the container 112 comprises a first edge 134 disposed externally with respect to the seating 25, by means of which to constrain the bag 32. The first edge 134 is conformed for example as a curl, lip or tongue, or in any case as an upside down "U", so as to define a groove 139 for a stable attachment of the margin 35 of the bag 32.

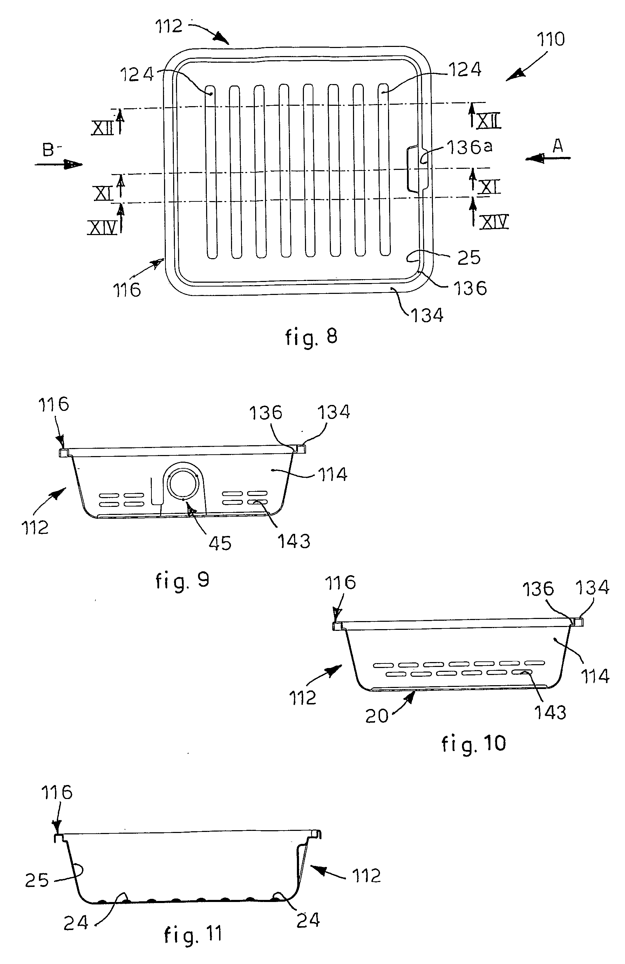

Moreover, each upper end 116 also comprises a second edge 136, disposed inside with respect to the first edge 134, between the latter and the seating 25 (figs. 8-14). The second edge 136 has the function of acting as a support for the lid 26 or 126 of the device 110. In particular, the second edge 136 has a substantially planar shape, to give a stable support for the edge 140 of the lid 26 or 126.

In this way the lid 26 or 126 is housed inside an internal peripheral seating defined by the second edge 136, and is sunk in the container 112, in a lowered position, substantially flush with the first edge 134. This has an advantageous holding function, preventing the lid 26, 126 from sliding laterally and uncoupling itself from the container 112.

On one of the cited lateral walls 114, as in the form of embodiment in figs. 1-5, the second aperture 42 is made, in which the rapid connector 45 is assembled with the functions already described. In particular, in some forms of embodiment, as can be seen in fig. 14, there is a shaped wall 114a inside the seating 25, to which the rapid connector 45 can be mechanically coupled.

The lid 126 in figs. 15 and 16 has a substantially rectangular shape, corresponding to that of the first aperture 18, and has, on at least one side, a positioning fin 141. In a mating manner, the second edge 136 has a hollow 136a, of a shape correlated to that of the positioning fin 141, so that the insertion of the latter into the hollow 136a defines a univocal positioning condition.

The lid 126 also has indicator elements, in this case arrows 131 made in one piece, which visually indicate the correct gripping and positioning direction of the lid 126 on the container 112 to the operator, and possibly rectilinear segments 132, to display the wrong insertion direction.

In the solution of the lid 126, indicator means 130 are provided to indicate, in an indirect manner by means of a code, for example alphanumeric and/or chromatic, the state of the instruments contained, in particular whether they are dirty or clean. The indicator means 130 provide a housing 130a in which an indicator or information disc is pivoted, for example with two sectors to indicate the conditions of dirtiness or cleanliness, for example a colored selector disc with two equal sectors, of which one is red (dirty) and one is green (clean). The housing 130a has a window or aperture 133, of a shape mating with that of the sectors of the disc. The rotation of the disc by the operator allows to position it so that one of the two sectors is visible through the window 113, depending on the state of the instrument.

The device 10, 110 according to the present invention is used according to the following procedure for the transport, washing and drying of a medical instrument 11, typically a flexible endoscope, before and after use, from the operating room or surgery to a washing station 50 and subsequent drying station 56 to be subsequently stored in a store room 59 and then re-used when needed (figs. 6 and 7).

The bag 32 is first attached to the first internal edge 34 of the container 12 of the transport device 10, or to the first external edge 134 of the container 112 of the device 110. The medical instrument 11, dirty after use, is inserted into the bag 32 inside the seating 25 of the container 12, 112 and the bag 32 is than closed by drawing the lace 37. The container 12 is then closed by the lid 26, 126. The indicator on the lid 26, 126, in the form of the signaler disc 30 or indicator means 130, is positioned on red.

The protruding elements 24 on the lower surface 22 of the bottom wall 20 prevent possible residual deposits or stagnations of biological materials or washing liquids from coming into contact with and adhering to the bag 32, thus preventing the formation of contaminated zones or preferential proliferation of pathogens on the bottom of the container 12, 112 (figs. 2 and 7).

The device 10, 110 is transported to a washing station 50, in particular comprising at least a pre-wash machine 52 and a wash machine 54, where the lid 26, 126 is removed and the bag 32 containing the medical instrument 11 is removed.

The medical instrument 11 is in turn removed from the bag 32 and is washed in the washing station 50, while the bag 32 is eliminated as indicated by the arrow W in fig. 7. In particular, a cleaning cycle is started that comprises a pre-wash step in the pre-wash machine 52 and a subsequent washing step in the appropriate wash machine 54. In the case of flexible endoscopes, normally no heat disinfection is carried out, but a chemical disinfection may be combined.

At the end of the washing step, the medical instrument 11 is removed from the wash machine 54 and deposited inside a new, clean bag 132 (fig. 7) which, as a preliminary, has already been attached to the first internal edge 34 of the container 12, or to the first external edge 134 of the container 112. Everything is then sent to a drying station 56 comprising a drying machine 57, conformed as a cupboard, into which the container 12, 112 is positioned, together with a plurality of other similar or comparable containers. The drying machine 57 is provided with means 55 to blow in air and with a first drying connector element 58 (fig. 7), through which the drying air is blown in at a desired temperature to the container 12, 112.

Before inserting the container 12, 112 into the drying machine 57, with the medical instrument 11 located in the bag 132, the internal connections 47 of the rapid connector 45 are fluidically connected to internal channels of the medical instrument 11 to be dried through a second connector element 13 provided on the medical instrument 11 (fig. 7). This operation is effected by lowering a desired portion of the bag 132 to allow communication and connection of the connections 47 and the second connector element 13. In fact the operator, advantageously when the container 12, 112 is still outside the drying machine 57, can easily maneuver the internal connections 47 through the first aperture 18 by acting on the medical instrument 11, also thanks to the flexibility of the bag 132 that is lowered by the amount necessary to reach the medical instrument 11 inside it, and not to interfere with or prevent the maneuver. In particular, part of the upper margin 35 of the bag 132 can be uncoupled from the first edge 34, 134, lowering a lip of material, so that the medical instrument 11 can be reached.

Then the connection of the internal connections 47 and the second connector element 13 is effected, keeping the medical instrument 11 always in the container 12, 112 used for transport.

Alternatively, the bag 132 can be temporarily removed, putting the medical instrument 11 to be dried directly into the container 12, 112. This prevents the bag 132 from obstructing the third apertures or slits 43, 143 through which the air passes, and also solves the problem of stagnating liquid which is collected by the bag 132, for example between the folds or on the bottom, which may not be completely dry.

Subsequently, when the container 12, 112 is located in the drying machine 57, the first connector element 58 is connected to the rapid connector 45, substantially automatically, always keeping the medical instrument 11 inside the same container 12, 112 used for transport, thus allowing the drying air to pass.

Subsequently, the blower means 55 are activated, for the drying, and blow sterile air through the first connector element 58.

The blower means 55 also blow sterile drying air directly into the container 12, 112 through the third apertures 43, in order to dry the external surface of the medical instrument 11.

If the lid 26, 126 has not been positioned to close the container 12, 112, the drying air advantageously also enters through the first aperture 18.

If, on the contrary, the lid 26, 126 has been positioned to close the container 12, 112, it is preferable to dispose the bag 132 in a lowered position in at least one portion, again exploiting the flexibility and deformability of the material of which it is made, for example disconnecting and lowering a portion thereof from the first edge 34, 134, so as to allow the air to enter into the bag 132 and to allow to dry the external surfaces of the medical instrument 11 too. Or, as we said, the bag 132 can be temporarily removed in the drying step and then again positioned in the container 12, 112. As we said, the inside of the medical instrument 11, for example the channels of a flexible endoscope, is dried by the air blown in by the first connector element 58.

After the drying step, the second connector element 13 is disconnected from the first connector element 58, the clean bag 132, if necessary repositioned if it had been temporarily removed for drying, is closed with the lace 37. The container 12, 112 is then re-closed with the lid 26, 126 and the indicator, in the form of the signaler disc 30 or indicator means 130, is positioned on green. The container 12, 112 can advantageously be inserted into another clean bag 232 to be transported to the store 59 (fig. 7).

For another use, the lid 26, 126 is opened and the new bag 132 is removed with inside it the clean medical instrument 11 which is in turn removed and used and then the cycle starts again as described above.

Modifications and variants may be made to the present invention, all of which come within the protected field as defined by the following claims.

## Claims

1. A combination to transport one or more medical instruments (11) both before and after they have been used, comprising a container (12, 112), a bag (32, 132) able to contain the medical instrument (11) and a lid (26, 126), said container (12, 112) comprising lateral walls (14, 114) to define an internal containing seating (25) and which have upper ends (16, 116) that delimit a first aperture (18) through which the bag (32, 132) is inserted into the seating (25) of the container (12), wherein said upper ends (16, 116) comprise a first edge inside (34) or outside (134) said seating (25) and shaped to cooperate with a mating upper margin (35) of said bag (32) so as to define a selectively releasable attachment of said bag (32, 132) and said container (12, 112), and a second edge (36), in a position outside (36) or respectively inside (136) said first edge (34, 134) with respect to said internal seating (25), the second edge (36, 136) being able to cooperate with said lid (26) in order to close said container (12, 112), wherein at least one of said lateral walls (14, 114) of said container (12) has a second aperture (42) that puts the outside in communication with the internal seating (25), the second aperture (42) being provided with a rapid connector (45) for the fluidic connection on one side to blower means (55) to blow in a drying fluid, and on the other side, by means of connectors (47), to the medical instrument (11).

2. A combination as in claim 1, **characterized in that** said first edge (34, 134) develops completely along the perimeter of the first aperture (18).

3. A combination as in claim 1, **characterized in that** said first edge (34,134) develops partly along the perimeter of the first aperture (18).

4. A combination as in claim 3, **characterized in that** said first edge (34, 134) is localized in desired different positions of said upper ends (16, 116) along the perimeter of the first aperture (18).

5. A combination as in any claim hereinbefore, **characterized in that** at least said first edge (34, 134) has a beveled conformation in a curl, tongue or lip.

6. A combination as in any claim hereinbefore, **characterized in that** said container (12, 112) also comprises a bottom wall (20) from which said lateral walls (14, 114) extend, and which includes a surface (22) inside said seating (25) from which protruding elements (24) arise, able to function as spacers to keep the bag (32, 132) distanced from the bottom wall (20) by a desired amount.

7. A combination as in any claim hereinbefore, **characterized in that** said bag (32, 132) can be selectively closed reversibly by means of closing means (37).

8. A combination as in any claim hereinbefore, **characterized in that** said lid (26, 126) is provided with indicator or display means (30) able to selectively indicate or display, directly or indirectly, the state at least of dirtiness or cleanliness of the medical instrument (11) contained.

9. A combination as in any claim hereinbefore, **characterized in that** said lid (26) comprises a peripheral portion (40) having an extension such as to completely cover the second edge (36) of the upper ends (16) of the lateral walls (14) of the container (12) when the lid (26) is in the closed position, so as to prevent contacts between potentially contaminated zones of the container (12) and the outside.

10. Container for one or more medical instruments (11) to be transported both before and after they have been used, comprising lateral walls (14, 114) to define an internal containing seating (25) and which have upper ends (16, 116) that delimit a first aperture (18) through which a bag (32, 132) containing said medical instruments (11) is inserted into the seating (25), said bag (32, 132) being associable with the upper ends (16, 116) of said lateral walls (14, 114), wherein said upper ends (16, 116) comprise a first edge inside (34) or outside (134) said seating (25) and shaped to cooperate with a mating upper margin (35) of said bag (32, 132) so as to define a selectively releasable attachment of said bag (32, 132) and said container (12), and a second edge in a position outside (36) or respectively inside (136) said first edge (34, 134) with respect to said internal seating (25), said second edge (36) being able to cooperate with a mating lid (26) in order to close said container (12, 112), wherein at least one of said lateral walls (14, 114) of said container (12) has a second aperture (42) which puts the outside in communication with the internal (25) seating, the second aperture (42) being provided with a rapid connector (45) for the fluidic connection on one side to blower means (55) to blow in a drying fluid, and on the other side, by means of connectors (47), to the medical instrument (11).

11. Method for drying one or more medical instruments (11) subjected to a cleaning cycle after use and inserted inside a container (12, 112) as in claim 10, used to transport said medical instruments (11) both before and after they have been used, comprising a step of positioning the medical instrument (11) inside a drying machine (57) of a drying station (56) where blower means (55) are able to blow in a drying fluid toward the medical instrument (11) contained in the container (12, 112) in order to dry at least the external surface thereof, said blower means also being connected by means of at least a connector element (5 8) to a mating second connector element (13) of the medical instrument (11) so as to blow in said drying fluid also inside the medical instrument (11), including the step of drying the medical instrument (11) positioned in the same container (12, 112) that is used for transport and wherein the connection between the first connector element (58) of the drying machine (57) and the second connector element (13) of the medical instrument (11) is effected keeping the medical instrument (11) inside the container (12, 112) and connecting the first connector element (58) to a rapid connector (45) associated with a corresponding lateral aperture (42) made in said container (12, 112), said rapid connector (45) providing connectors (47) inside the container (12, 112) for connection to the second connector element (13) of the medical instrument (11).

12. Method to transport one or more medical instruments (11) both before and after they have been used, by means of a combination as in any claim from 1 to 9,
comprising the following steps:
- a first step in which a bag (32) is positioned in the seating (25) of the container (12, 112) and the upper margin (35) of said bag (32) is attached to the first internal edge (34) or external edge (134), also determining the opening thereof;
- a second step in which the dirty medical instrument is disposed in the bag (32), the bag is closed in reversible manner and the lid (26, 126) is positioned so as to close the container (12, 112) in cooperation with the second external edge (36) or internal edge (136) respectively;
- a third step in which the container (12, 112) is transported to a washing station (50) where the lid (26, 126) is opened, the dirty medical instrument (11) is removed so as to be subjected to a cleaning cycle, the used bag (32) is eliminated and at the same time a new bag (132) is positioned in the seating (25) of the container (12, 112), as in the first step;
- a fourth step in which the medical instrument (11) is positioned in the clean new bag (132) and is transported to a drying station (56) where it undergoes drying in accordance with the drying method as in claim 11;
- a fifth step in which the new bag (132) containing the dried medical instrument (11) is closed in a reversible manner and the lid (26, 126) is positioned so as to close the container (12, 112) as in the second step, making the medical instrument (11) available for another use.

## Patentansprüche

1. Eine Kombination zum Transportieren von einem oder mehreren medizinischen Instrumenten (11) sowohl bevor als auch nach dem diese verwendet wurden, aufweisend einen Behälter (12, 112), einen Beutel (32, 132), der in der Lage ist, das medizinische Instrument (11) aufzunehmen, und einen Deckel (26, 126), wobei der Behälter (12, 112) Seitenwände (14, 114) aufweist, um eine innere Behälteraufnahme (25) zu definieren, und wobei diese obere Enden (16, 116) aufweisen, die eine erste Öffnung (18) begrenzen, durch die hindurch der Beutel (32, 132) in die Aufnahme (25) des Behälters (12) eingesetzt wird, wobei die oberen Enden (16, 116) aufweisen: einen ersten Rand innerhalb (34) oder außerhalb (134) der Aufnahme (25) und in einer Form, um mit einem passenden oberen Rand (35) des Beutels (32) zusammenzuwirken, um eine wahlweise lösbare Befestigung des Beutel (32, 132) und des Behälters (12, 112) zu definieren, und einen zweiten Rand (36) in einer Position außerhalb (36) bzw. innerhalb (136) von dem ersten Rand (34, 134) bezüglich der inneren Aufnahme (25), wobei der zweite Rand (36, 136) in der Lage ist, mit dem Deckel (26) zusammenzuwirken, um den Behälter (12, 112) zu schließen, wobei mindestens eine der Seitenwände (14, 114) des Behälters (12) eine zweite Öffnung (42) hat, die die Außenseite mit der inneren Aufnahme (25) in Kommunikation bringt, wobei die zweite Öffnung (42) mit einem Schnellverbinder (45) für die Fluidverbindung auf einer Seite mit Gebläsemitteln (55) zum Einblasen eines Trocknungsfluids, und auf der anderen Seite mittels Verbindungsstücken (47) mit dem medizinischen Instrument (11) versehen ist.

2. Eine Kombination wie in Anspruch 1, **dadurch gekennzeichnet, dass** der erste Rand (34, 134) vollständig entlang dem Umfang der ersten Öffnung (18) ausgebildet ist.

3. Eine Kombination wie in Anspruch 1, **dadurch gekennzeichnet, dass** der erste Rand (34, 134) teilweise entlang dem Umfang der ersten Öffnung (18) ausgebildet ist.

4. Eine Kombination wie in Anspruch 3, **dadurch gekennzeichnet, dass** der erste Rand (34, 134) in gewünschten unterschiedlichen Positionen der oberen Enden (16, 116) entlang dem Umfang der ersten Öffnung (18) positioniert ist.

5. Eine Kombination wie in irgendeinem vorhergehenden Anspruch, **dadurch gekennzeichnet, dass** mindestens der erste Rand (34, 134) eine abgeschrägte Gestaltung als Bördelung, Zunge oder Lippe hat.

6. Eine Kombination wie in irgendeinem vorhergehenden Anspruch, **dadurch gekennzeichnet, dass** der Behälter (12, 112) ferner eine untere Wand (20) aufweist, von der aus sich die Seitenwände (14, 114) erstrecken, und die eine Fläche (22) innerhalb der Aufnahme (25) aufweist, aus der hervorstehende Elemente (24) hervorgehen, die in der Lage sind, als Abstandshalter zu funktionieren, um den Beutel (32, 132) in einem gewünschten Maße im Abstand von der unteren Wand (20) zu halten.

7. Eine Kombination wie in irgendeinem vorhergehenden Anspruch, **dadurch gekennzeichnet, dass** der Beutel (32, 132) mittels Schließmitteln (37) wahlweise reversibel geschlossen werden kann.

8. Eine Kombination wie in irgendeinem vorhergehenden Anspruch, **dadurch gekennzeichnet, dass** der Deckel (26, 126) mit Hinweis- oder Anzeigemitteln (30) versehen ist, die in der Lage sind, den Zustand von mindestens einer Verschmutzung oder Sauberkeit des enthaltenen medizinischen Instruments (11) wahlweise direkt oder indirekt anzugeben oder anzuzeigen.

9. Eine Kombination wie in irgendeinem vorhergehenden Anspruch, **dadurch gekennzeichnet, dass** der Deckel (26) einen Umfangsabschnitt (40) aufweist, der eine Erweiterung hat, um den zweiten Rand (36) der oberen Enden (16) der Seitenwände (14) des Behälters (12) vollständig zu bedecken, wenn sich der Deckel (26) in der Schließposition befindet, um Kontakte zwischen möglicherweise kontaminierten Bereichen des Behälters (12) und dem Äußeren zu verhindern.

10. Behälter für ein oder mehrere medizinische Instrumente (11) zum Transportieren sowohl bevor als auch nachdem diese verwendet wurden, aufweisend Seitenwände (14, 114), um eine innere Behälteraufnahme (25) zu definieren, und die obere Enden (16, 116) aufweisen, die eine erste Öffnung (18) begrenzen, durch die hindurch ein Beutel (32, 132), der die medizinischen Instrumente (11) enthält, in die Aufnahme (25) eingesetzt ist, wobei der Beutel (32, 132) mit den oberen Enden (16, 116) der Seitenwände (14, 114) verbindbar ist, wobei die oberen Enden (16, 116) aufweisen: einen ersten Rand innerhalb (34) oder außerhalb (134) der Aufnahme (25) und in einer Form, um mit einem passenden oberen Rand (35) des Beutels (32, 132) zusammenzuwirken, um eine wahlweise lösbare Befestigung des Beutel (32, 132) und des Behälters (12) zu definieren, und einen zweiten Rand in einer Position außerhalb (36) bzw. innerhalb (136) des ersten Randes (34, 134) bezüglich der inneren Aufnahme (25), wobei der zweite Rand (36) in der Lage ist, mit einem passenden Deckel (26) zusammenzuwirken, um den Behälter (12, 112) zu verschließen, wobei mindestens eine der Seitenwände (14, 114) des Behälters (12) eine zweite Öffnung (42) hat, die das Äußere mit der inneren (25) Aufnahme in Kommunikation bringt, wobei die zweite Öffnung (42) mit einem Schnellverbinder (45) für die Fluidverbindung auf einer Seite mit Gebläsemitteln (55) zum Einblasen eines Trocknungsfluids und auf der anderen Seite mittels Verbindungsstücken (47) mit dem medizinischen Instrument (11) versehen ist.

11. Verfahren zum Trocknen von einem oder mehreren medizinischen Instrumenten (11), die nach der Verwendung einem Reinigungszyklus unterzogen werden und innerhalb eines Behälters (12, 112) wie in Anspruch 10 eingesetzt sind, der zum Transportieren der medizinischen Instrumente (11) sowohl vor als auch nach ihrer Verwendung verwendet wird, aufweisend einen Schritt des Positionierens des medizinischen Instruments (11) innerhalb eines Trockners (57) einer Trocknungsstation (56), wo Gebläsemittel (55) in der Lage sind, ein Trocknungsfluid in Richtung des medizinischen Instruments (11) einzublasen, das in dem Behälter (12, 112) enthalten ist, um zumindest die Außenfläche davon zu trocknen, wobei die Gebläsemittel ferner mittels mindestens eines Verbindungselements (58) mit einem passenden zweiten Verbindungselement (13) des medizinischen Instruments (11) verbunden sind, um das Trocknungsfluid auch in das medizinische Instrument (11) hineinzublasen, aufweisend den Schritt des Trocknens des medizinischen Instruments (11), das in demselben Behälter (12, 112) positioniert ist, der für den Transport verwendet wird, und wobei die Verbindung zwischen dem ersten Verbindungselement (58) des Trockners (57) und dem zweiten Verbindungselement (13) des medizinischen Instruments (11) erfolgt, wobei das medizinische Instrument (11) innerhalb des Behälters (12, 112) gehalten wird und das erste Verbindungselement (58) mit einem Schnellverbinder (45) verbunden wird, der einer korrespondierenden seitlichen Öffnung (42) zugeordnet ist, die in dem besagten Behälter (12, 112) ausgebildet ist, wobei der besagte Schnellverbinder (45) Verbindungsstücke (47) innerhalb des Behälters (12, 112) zum Verbinden mit dem zweiten Verbindungselement (13) des medizinischen Instruments (11) bereitstellt.

12. Verfahren zum Transportieren von einem oder mehreren medizinischen Instrumenten (11) sowohl bevor als auch nachdem diese verwendet wurden, mittels einer Kombination wie in irgendeinem Anspruch 1 bis 9, aufweisend die folgenden Schritte:
- einen ersten Schritt, in welchem ein Beutel (32) in der Aufnahme (25) des Behälters (12, 112) positioniert wird und der obere Rand (35) des Beutels (32) an dem ersten inneren Rand (34) oder äußeren Rand (134) angebracht wird, der auch die Öffnung davon bestimmt,
- einen zweiten Schritt, in welchem das verschmutzte medizinische Instrument in dem Beutel (32) angeordnet wird, der Beutel auf reversible Weise geschlossen wird und der Deckel (26, 126) positioniert wird, um den Behälter (12, 112) in Zusammenwirkung mit dem zweiten äußeren Rand (36) bzw. inneren Rand (136) zu verschließen,
- einen dritten Schritt, in welchem der Behälter (12, 112) zu einer Waschstation (50) transportiert wird, wo der Deckel (26, 126) geöffnet wird, das verschmutzte medizinische Instrument (11) herausgenommen wird, um einem Reinigungszyklus unterzogen zu werden, der gebrauchte Beutel (32) beseitigt wird und gleichzeitig ein neuer Beutel (132) in der Aufnahme (25) des Behälters (12, 112) positioniert wird, wie in dem ersten Schritt,
- einen vierten Schritt, in welchem das medizinische Instrument (11) in dem sauberen neuen Beutel (132) positioniert wird und zu einer Trocknungsstation (56) transportiert wird, wo es einem Trocknen gemäß dem Trocknungsverfahren wie in Anspruch 11 unterzogen wird,
- einen fünften Schritt, in welchem der neue Beutel (132), der das getrocknete medizinische Instrument (11) enthält, auf reversible Weise verschlossen wird und der Deckel (26, 126) positioniert wird, um den Behälter (12, 112) wie in dem zweiten Schritt zu verschließen, wobei das medizinische Instrument (11) für eine weitere Verwendung verfügbar gemacht wird.

## Revendications

1. Combinaison pour transporter un ou plusieurs instruments médicaux (11), avant et après leur emploi, comprenant un contenant (12, 112), un sac (32, 132) pour contenir l'instrument médical (11) et un couvercle (26, 126), ledit contenant (12, 112) comprenant des parois latérales (14, 114) définissant un logement de confinement interne (25) et possédant des extrémités supérieures (16, 116) qui délimitent une première ouverture (18) par laquelle le sac (32, 132) est inséré dans le logement (25) du contenant (12), dans laquelle lesdites extrémités supérieures (16, 116) comprennent un premier bord à l'intérieur (34) ou à l'extérieur (134) dudit logement (25) et sont formées de manière à coopérer avec une marge supérieure complémentaire (35) dudit sac (32) pour définir un accouplement sélectivement libérable dudit sac (32, 132) et dudit contenant (12, 112), et un second bord, situé respectivement à l'extérieur (36) ou à l'intérieur (136) dudit premier bord (34, 134) par rapport audit logement interne (25), le second bord (36, 136) pouvant coopérer avec ledit couvercle (26) afin de fermer ledit contenant (12, 112), dans laquelle au moins une desdites parois latérales (14, 114) dudit contenant (12) possède une seconde ouverture (42) qui met le logement interne (25) en communication avec l'extérieur, la seconde ouverture (42) étant pourvue d'un connecteur rapide (45) pour établir une communication fluidique avec des moyens soufflants (55) sur un côté pour souffler un fluide de séchage à l'intérieur, et avec l'instrument médical (11), au moyen de connecteurs (47), sur l'autre côté.

2. Combinaison selon la revendication 1, **caractérisée en ce que** ledit premier bord (34, 134) s'étend entièrement le long du périmètre de la première ouverture (18).

3. Combinaison selon la revendication 1, **caractérisée en ce que** ledit premier bord (34, 134) s'étend en partie le long du périmètre de la première ouverture (18).

4. Combinaison selon la revendication 3, **caractérisée en ce que** ledit premier bord (34, 134) est localisé en positions différentes souhaitées desdites extrémités supérieures (16, 116) le long du périmètre de la première ouverture (18).

5. Combinaison selon n'importe laquelle des revendications précédentes, **caractérisée en ce qu'**au moins ledit premier bord (34, 134) présente une conformation biseautée en boucle, languette ou lèvre.

6. Combinaison selon n'importe laquelle des revendications précédentes, **caractérisée en ce que** ledit contenant (12, 112) comprend également une paroi de fond (20) de laquelle s'étendent lesdites parois latérales (14, 114), et laquelle comprend une surface (22) à l'intérieur dudit logement (25) de laquelle s'élèvent des éléments en saillie (24), pouvant servir d'écarteurs pour maintenir le sac (32, 132) à une certaine distance de la paroi de fond (20).

7. Combinaison selon n'importe laquelle des revendications précédentes, **caractérisée en ce que** ledit sac (32, 132) peut être sélectivement fermé de façon réversible par des moyens de fermeture (37).

8. Combinaison selon n'importe laquelle des revendications précédentes, **caractérisée en ce que** ledit couvercle (26, 126) est pourvu de moyens indicateurs ou afficheurs (30) pouvant indiquer ou afficher sélectivement, de façon directe ou indirecte, au moins l'état de saleté ou de propreté de l'instrument médical (11) contenu.

9. Combinaison selon n'importe laquelle des revendications précédentes, **caractérisée en ce que** ledit couvercle (26) comprend un partie périphérique (40) ayant une telle extension à couvrir complètement le second bord (36) des extrémités supérieures (16) des parois latérales (14) du contenant (12) quand le couvercle (26) est en position fermée, de manière à empêcher tout contact entre des zones potentiellement contaminées du contenant (12) et extérieur.

10. Contenant pour un ou plusieurs instruments médicaux (11), à transporter avant et après leur emploi, comprenant des parois latérales (14, 114) définissant un logement de confinement interne (25) et possédant des extrémités supérieures (16, 116) qui délimitent une première ouverture (18) par laquelle un sac (32, 132) est inséré dans le logement (25), ledit sac (32, 132) étant associable aux extrémités supérieures (16, 116) desdites parois latérales (14, 114), dans lequel lesdites extrémités supérieures (16, 116) comprennent un premier bord à l'intérieur (34) ou à l'extérieur (134) dudit logement (25) et sont formées de manière à coopérer avec une marge supérieure conjuguée (35) dudit sac (32, 132) pour définir un accouplement sélectivement libérable dudit sac (32, 132) et dudit contenant (12), et un second bord, situé respectivement à l'extérieur (36) ou à l'intérieur (136) dudit premier bord (34, 134) par rapport audit logement interne (25), ledit second bord (36) pouvant coopérer avec un couvercle conjugué (26) afin de fermer ledit contenant (12, 112), dans lequel au moins une desdites parois latérales (14, 114) dudit contenant (12) possède une seconde ouverture (42) qui met le logement interne (25) en communication avec l'extérieur, la seconde ouverture (42) étant pourvue d'un connecteur rapide (45) pour établir une communication fluidique avec des moyens soufflants (55) sur un côté pour souffler un fluide de séchage à l'intérieur, et avec l'instrument médical (11), au moyen de connecteurs (47), sur l'autre côté.

11. Procédé pour sécher un ou plusieurs instruments médicaux (11) soumis à un cycle de nettoyage après l'emploi et insérés dans un contenant (12, 112) selon la revendication 10, utilisé pour transporter lesdits instruments médicaux (11) avant et après leur emploi, comprenant une étape de positionnement de l'instrument médical (11) à l'intérieur d'une sécheuse (57) d'une station de séchage (56) où des moyens soufflants (55) peuvent souffler un fluide de séchage à l'intérieur vers l'instrument médical (11) contenu dans le contenant (12, 112) pour sécher au moins la surface extérieure de celui-ci, lesdits moyens soufflants étant également reliés au moyen d'au moins un élément connecteur (58) à un second élément connecteur (13) conjugué de l'instrument médical (11) pour souffler ledit fluide de séchage aussi bien à l'intérieur de l'instrument médical (11), séchage de l'instrument médical (11) positionné dans le même contenant (12, 112) employé pour le transport et dans lequel la liaison entre le premier élément connecteur (58) de la sécheuse (57) et le second élément connecteur (13) de l'instrument médical (11) est effectuée en maintenant l'instrument médical (11) à l'intérieur du contenant (12, 112) et reliant le premier élément connecteur (58) à un connecteur rapide (45) associé à une ouverture latérale correspondante (42) réalisée dans ledit contenant (12, 112), ledit connecteur rapide (45) comportant des connecteurs (47) à l'intérieur du contenant (12, 112) pour la liaison au second élément connecteur (13) de l'instrument médical (11).

12. Procédé pour transporter un ou plusieurs instruments médicaux (11) avant et après leur emploi au moyen d'une combinaison selon n'importe laquelle des revendications 1 à 9, comprenant les étapes suivantes :
- une première étape dans laquelle un sac (32) est positionné dans le logement (25) du contenant (12, 112) et la marge supérieure (35) dudit sac (32) est fixé au premier bord intérieur (34) ou extérieur (134), ce qui cause aussi l'ouverture de celui-ci ;
- une deuxième étape dans laquelle l'instrument médical sale est placé dans le sac (32), le sac est fermé de manière réversible et le couvercle (26, 126) est positionné de façon à fermer le contenant (12, 112) en coopération avec respectivement le second bord extérieur (36) ou intérieur (136) ;
- une troisième étape dans laquelle le contenant (12, 112) est transporté vers une station de lavage (50) où le couvercle (26, 126) est ouvert, l'instrument médical sale (11) est enlevé pour être soumis à un cycle de nettoyage, le sac usé (32) est éliminé et en même temps un nouveau sac (132) est positionné dans le logement (25) du contenant (12, 112) comme dans la première étape ;
- une quatrième étape dans laquelle l'instrument médical (11) est positionné dans le nouveau sac propre (132) et transporté vers une station de séchage (56) où il est séché avec le procédé de séchage selon la revendication 11 ;
- une cinquième étape dans laquelle le nouveau sac (132) contenant l'instrument médical séché (11) est fermé de façon réversible et le couvercle (26, 126) est positionné de manière à fermer le contenant (12, 112) comme dans la deuxième étape, pour rendre l'instrument médical (11) disponible pour une nouvelle utilisation.
